# EUROPEAN PATENT APPLICATION

(11) **EP 3 255 051 A1**
(43) Date of publication of application: **13.12.2017**
(21) Application number: 16173641.8
(22) Date of filing: 09.06.2016
(51) Int. Cl.: C07D 489/02

(54) **SUPPORTED METAL CATALYST FOR THE PRODUCTION OF HYDROCODON AND HYDROMORPHON**

(71) Applicant: Siegfried AG, 4800 Zofingen (CH)
(72) Inventor: Weber, Beat, 4800 Zofingen (CH); Spälti, Michael, 8753 Mollis (CH)
(74) Representative: Harms, Guido

(57) **Abstract**

The present invention relates to the process for the manufacture of hydrocodone or hydromorphone from their enol derivatives codeine and morphine respectively. Particularly, the invention discloses a metal catalyst that is used in low amount, leads to high yields and can easily be reused.

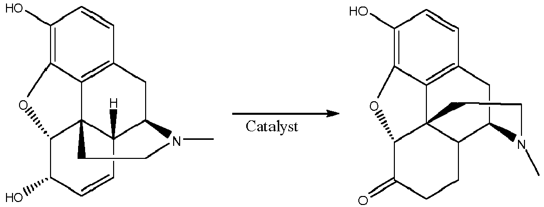

## Description

The present invention relates to the process for the manufacture of hydrocodone or hydromorphone from their enol derivatives codeine and morphine respectively. Particularly, the invention discloses a metal catalyst that is used in low amount, leads to high yields and can easily be reused.

### State of the art

Hydrocodone and Hydromorphone are widely used as active pharmaceutical ingredients. Hydromorphone is a derivative of morphine and can also be synthesized starting from morphine. In the same way, hydrocodone may be synthesized form codeine. Hydromorphone and hydrocodone are opioid analgesics that are commonly used in therapy.

Hydromorphone is synthesized from Morphine, a natural product.

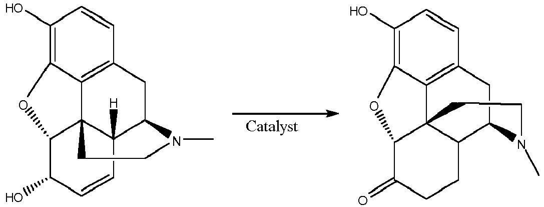

For this reaction, a variety of catalysts have been developed. Knoll has described the use of platin catalysts in this reaction (DE617238; DE607931; DE623821; DE365683; DE380919). Platin has been used as homogeneous catalysts (as PdCl₂ in hydrochloric acid, DE365683) as well as heterogenic catalysts for this reaction (DE607931, Expl. 3; DE623821, Expl. 1; DE617238, Expl.3). However, the yields are low and the amount of catalyst used is at a level of between 10 and 100 %.

Johnson Matthey has found a possibility to use catalysts with bidentate ligands in a homogenous catalysis. As the central atom in the catalysis the following metals were selected: Rh, Pd, Pt, Ir, or Fe (EP0915884B1). The reported yield is 35 %. The reaction requires Nitrogen atmosphere and anhydrous conditions. Also other researches have published reactions under homogenous conditions, using a Wilkinson catalyst (US6946556). However, the need of inert atmosphere and low yields show that the reaction can be improved.

The most promising reactions using homogeneous catalysts are shown in US7323565, using Wilkinson catalyst and other homogenous catalysts with aminophosphine ligands. These reactions, although showing high yields, suffer from the need of inert atmosphere and relatively expensive catalyst complexes.

In WO2005100361 Zentiva shows the conversion of morphine to hydromorphone using a combination of a soluble catalyst (PdCl) and a supported catalyst (Ru on C). In a two-step synthesis, hydromorphone is isolated in 75 % Yield. This might lead to sufficient yields, but the combination of catalysts always incorporates the risk of the loss of catalyst, because it might not be possible to recycle the catalyst. Furthermore the costs for the preparation are higher than for only using one catalyst.

The use of homogeneous catalysis often leads to products that contain a substantial amount of residual metal unless separated from the reaction mixture by means of scavengers (see WO2011137086).

WO2011035276 describes a way of manufacturing hydromorphone from morphine using a heterogeneous non-supported Ruthenium catalyst. The inventors utilize around 10 mol % of the catalyst, calculated on the amount of unsaturated ketone. Unfortunately, the application does not show how to isolate the reaction product from the reaction mixture. Therefore, the yield described is only theoretical and not for industrial use. Furthermore, the description proposes that the reaction may be conducted in water and no protective gas may be used, but in the examples the reaction is conducted using ethanol and also using a protective gas.

### Description of the invention

The state of the art shows that the conversion of morphine to hydromorphone is possible. However, there is a lack of a suitable catalyst that allows a reaction that is easy to handle, safe and that shows a high conversion rate of morphine to hydromorphone.

Summarizing, there is a need of improvement of the reaction conditions. It would be beneficial to find a catalyst that allows the reaction in a non-flammable solvent in order to prevent burn and also to minimize the risk of an explosion. Furthermore, it is the goal to find reaction conditions without the need of a protecting gas like nitrogen or argon, since a reaction in industrial scale is always easier to handle when the reaction may be performed under ambient conditions.

Hydrogen is often used in the conversion of morphine to hydromorphone, predominantly when a two-step approach is used. But as hydrogen is flammable and may cause explosions, it is not part of the ideal reaction conditions.

We have now found a way to prepare hydromorphone and hydrocodone from morphine and codeine, respectively using a heterogeneous catalyst on a solid support.
Heterogeneous catalysis means in the context of this invention that the catalyst is insoluble, i.e. not soluble in the solvent or solvent mixture used for the reaction. It means further that the metal is used (when in the reaction mixture) as elemental metal, in an average oxidation state of zero, distributed on a solid, insoluble support. The catalyst used for this invention is not part of a ligand complex. The support may be chosen from various kinds of zeolite, carbon, alumina, aluminum oxide, magnesium chloride, potassium oxide, silica, Barium sulfate, Titanium oxide, magnesium oxide and others.

The invention involves a process for the preparation of a compound with a structure of formula II, obtained by reacting a compound with a structure of formula I in a way that a compound of formula II is produced, comprising that the reaction is conducted in the presence of a heterogenous catalyst on a solid support. 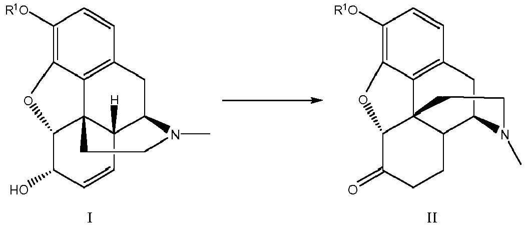 wherein R1 is selected from CH₃ and Hydrogen.

Furthermore it could be shown that the catalyst can easily be reused without drying or other workup.

Whenever an amount of catalyst in % occurs in this description, it is clear to the person skilled in the art that the percentage is calculated as amount of metal catalyst to amount of morphine or codeine as a molar ratio.

It has been found that the best yields are achieved when using ruthenium or rhodium as the catalyst. Preferred catalyst is ruthenium as it is cheaper compared to rhodium.

In a preferred embodiment of the invention the catalyst is supported on carbon or aluminum oxide. Other supporting materials are also possible, but did not result in as high yields as carbon and aluminum oxide. The most preferred support of the catalyst is an aluminum oxide support.

The advantage of using a supported catalyst is not only the isolation of the catalyst after the reaction, but also the low amount of residual catalyst in the product. The product as produced by the inventive method has less than 10 ppm of residual catalyst.

We have surprisingly found that for the invention it is not necessary to use any cosolvent other than water. Whereas a cosolvent does not reduce the yield or the conversion rate, but has some disadvantages in terms of workup of the reaction mixture and the safety measures during the reaction in large scale. When using an organic flammable solvent like ethanol or isopropanol in an appropriate amount, it is mandatory to use a protecting gas for safety reasons.

However, when using the reaction conditions of the current invention, it is not necessary to involve any cosolvents. The reaction is therefore preferably conducted in water or in a mixture of water and one or more cosolvents whereas the water content in the solvent mixture is preferably at least 90 %. Keeping these conditions, there is furthermore surprisingly no need of using a protecting gas for the reaction. It is also noted that in the synthesis no Hydrogen is needed. In the context of the invention this is also described as ambient atmosphere conditions. Ambient atmosphere conditions shall mean in the context of this invention that no inert gas and no Hydrogen is used and that no pressure is applied to the reaction vessel, i.e. the reaction is conducted under air.

This makes the reaction more safe, easier to handle and cheaper.

In a preferred embodiment of the invention, the reaction is conducted using only one type of metal catalyst. Using a single catalyst on a solid support has the advantage over the state of the art that only 0.2 - 10 mol % of the metal catalyst need to be used, and that the catalyst is easily separated from the reaction mixture by filtration.

The use of only one single catalyst allows furthermore recycling the catalyst. It was found that the catalyst can easily be reused.

### Examples

### Example 1 (reference example)

### Synthesis of Hydromorphone from Morphine with complexed ruthenium (homogeneous catalysis)

Water (1.5 ml) and concentrated sulfuric acid (0.3g, 0.16 mL, 0.003 mol, 0.85 eq.) were filled into a flask equipped with nitrogen inlet. Morphine (1.0g, 0.0035 mol, 1.0 eq.) was added and the flask was flushed with nitrogen.

Dichlor(pentamethylcyclopentadienyl)ruthenium polymer (0.0053g, 0.0000175 mol, 0.5 mol%) and Ethanol (0.5 mL) were added and the mixture heated under reflux.

The reaction mixture was diluted with water (5 ml) and the catalyst was separated by filtration. The pH of the solution was adjusted to 7.5 and the product was separated by filtration.

After chromatographic purification 849 mg of Hydromorphone has been isolated. (2,9 mmol, 85 %) Purity by HPLC: 99 %

### Example 2

### Preparation of Hydromorphone from Morphine using a supported ruthenium catalyst

Water (6 ml) and concentrated sulfuric acid (0.875g, 0.475 mL, 0.0089 mol, 0.85 eq.) were filled into a flask with reflux condenser. Morphine (3.0 g, 0.0105 mol, 1.0 eq.) und Ru/Alox (0.424g, 0.00021 mol, 2 mol%) were added and the mixture was heated under reflux.

After completion of the reaction (monitored by TLC), water was added to the reaction mixture (15 ml) and the mixture was filtered at 70 °C.

After adjustment of the pH to 7.5, the crude product could be separated by filtration. It was dissolved in methanol (40 ml), and activated charcoal (0.2 g, NORIT SUPER CAP) and Cellflock (0.2g) were added. The mixture was stirred for 20 minutes at 60 °C and filtered hot. After evaporation of the solvent, the product could be isolated as a white solid. After chromatographic purification 2,4 g of hydromorphone was isolated. (8,4 mmol, 80 %). Purity by HPLC: 99.5 %

### Example 3

### Preparation of Hydromorphone from Morphine using a supported rhodium catalyst

Water (6 ml) and concentrated sulfuric acid (0.875g, 0.475 mL, 0.0089 mol, 0.85 eq.) were filled into a flask with reflux condenser. Morphine (3.0 g, 0.0105 mol, 1.0 eq.) und Rh/Alox (0.108g, 0.000053 mol, 0,5 mol%) were added and the mixture was heated under reflux.

After completion of the reaction (monitored by TLC), water was added to the reaction mixture (15 ml) and the mixture was filtered at 70 °C.

After adjustment of the pH to 7.5, the crude product could be separated by filtration. It was dissolved in methanol (40 ml), and activated charcoal (0.2 g, NORIT SUPER CAP) and Cellflock (0.2g) were added. The mixture was stirred for 20 minutes at 60 °C and filtered hot. After evaporation of the solvent, the product could be isolated as a white solid. After chromatographic purification 832 mg of hydromorphone was isolated. (8,3 mmol, 78 %). Purity by HPLC: 99.0 %

### Example 4

### Preparation of Hydrocodone from Codeine using a supported ruthenium catalyst

Water (6 ml) and concentrated sulfuric acid (0.875g, 0.475 mL, 0.0089 mol, 0.85 eq.) were filled into a flask with reflux condenser. Codeine (3.1 g, 0.0104 mol, 1.0 eq.) und Ru/Alox (0.414g, 0,21 mmol, 2 mol%) were added and the mixture was heated under reflux.

After completion of the reaction (monitored by TLC), water was added to the reaction mixture (15 ml) and the mixture was filtered at 70 °C.

After adjustment of the pH to 7.5, the crude product could be separated by filtration. It was dissolved in methanol (40 ml), and activated charcoal (0.2 g, NORIT SUPER CAP) and Cellflock (0.2g) were added. The mixture was stirred for 20 minutes at 60 °C and filtered hot. After evaporation of the solvent, the product could be isolated as a white solid. After chromatographic purification 2420 mg of hydrocodone was isolated. (8.1 mmol, 78 %). Purity by HPLC: 99 %

### Example 5

### Preparation of Hydromorphone from Morphine using a reused supported ruthenium catalyst

Water (6 ml) and concentrated sulfuric acid (0.875g, 0.475 mL, 0.0089 mol, 0.85 eq.) were filled into a flask with reflux condenser. Morphine (3.0 g, 0.0105 mol, 1.0 eq.) und Ru/Alox (as separated from Example 2) were added and the mixture was heated under reflux.

After completion of the reaction (monitored by TLC), water was added to the reaction mixture (15 ml) and the mixture was filtered at 70 °C.

After adjustment of the pH to 8.5, the crude product could be separated by filtration. It was dissolved in methanol (40 ml), and activated charcoal (0.2 g, NORIT SUPER CAP) and Cellflock (0.2g) were added. The mixture was stirred for 20 minutes at 60 °C and filtered hot. After evaporation of the solvent, the product could be isolated as a white solid. After chromatographic purification 2,3 g of hydromorphone was isolated. (8,1 mmol, 77 %). Purity by HPLC: 99.0 %

## Claims

1. Process for the preparation of a compound with a structure of formula II, comprising the reaction of a compound with a structure of formula I, to give a compound of formula II, comprising that the reaction is conducted in the presence of a heterogeneous catalyst on a solid support and wherein the reaction is conducted under ambient atmosphere conditions 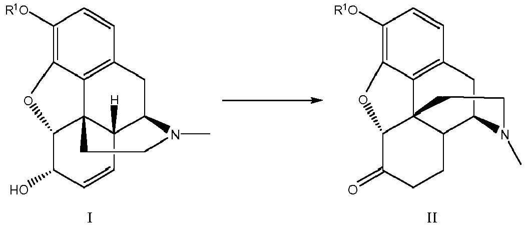 wherein R¹ is selected from CH₃ and Hydrogen.

2. Process of claim 1 using Ruthenium or Rhodium as the catalyst.

3. Process of claim 2 using Ruthenium as the catalyst.

4. Process of one of the preceding claims wherein the catalyst is supported on carbon or aluminum oxide.

5. Process of claim 4 wherein the support of the catalyst is an aluminum oxide support.

6. Process of one of the preceding claims wherein the reaction is conducted in the presence of water.

7. Process of one of the preceding claims wherein only one type of catalyst is used during the reaction.
